# EUROPEAN PATENT SPECIFICATION

(11) **EP 3 646 855 B1**
(45) Date of publication and mention of the grant of the patent: **01.05.2024**
(21) Application number: 18822758.1
(22) Date of filing: 29.06.2018
(51) Int. Cl.: A61K 9/16, A61K 31/4439, A61K 9/20, A61K 9/28

(54) **ORAL SOLID PREPARATION COMPOSITION COMPRISING PROTON PUMP INHIBITOR, ORAL SOLID PREPARATION COMPRISING SAME, AND PREPARATION METHOD THEREFOR**
ORALE FESTE ZUBEREITUNGSZUSAMMENSETZUNG MIT PROTONENPUMPENHEMMER, ORALE FESTE ZUBEREITUNG DAMIT UND HERSTELLUNGSVERFAHREN DAFÜR
COMPOSITION POUR PRÉPARATION SOLIDE À USAGE ORAL COMPRENANT UN INHIBITEUR DE POMPE À PROTONS, PRÉPARATION SOLIDE À USAGE ORAL LA COMPRENANT, ET SON PROCÉDÉ DE PRÉPARATION

(30) Priority: 30.06.2017 KR 20170083891; 30.06.2017 KR 20180006687
(43) Date of publication of application: 06.05.2020
(73) Proprietor: LOTTE Fine Chemical Co., Ltd., Nam-gu, Ulsan 44714 (KR)
(72) Inventor: SHIN, Kwang Il, Gunpo-si Gyeonggi-do 15804 (KR); LEE, Sang Youb, Incheon 21994 (KR)
(74) Representative: Michalski Hüttermann & Partner Patentanwälte mbB
(86) International application number: PCT/KR2018/007394
(87) International publication number: WO 2019/004770

(56) References cited:
- EP-A1- 2 929 885
- CN-A- 101 711 753
- CN-A- 102 772 387
- KR-A- 870 009 717
- KR-A- 20070 094 053
- KR-A- 20130 115 593
- KR-B1- 100 479 637
- KR-B1- 101 845 665
- US-A1- 2013 273 171
- HE, WEI et al.: "Influences of Sodium Carbonate on Physicochemical Properties of Lansoprazole in Designed Multiple Coating Pellets", AAPS PharmSciTech, vol. 11, no. 3, 2010, pages 1287-1293, XP055592405,

## Description

### Technical Field

The present disclosure relates to an oral solid preparation composition including a proton pump inhibitor, an oral solid preparation including the same, and a method of preparing the same, and more particularly to an oral solid preparation composition including an appropriate amount of a basic additive together with a proton pump inhibitor or a pharmaceutically acceptable salt thereof and, thus, exhibiting excellent stability with a significant reduction in the production of related substances; an oral solid preparation including the same; and a method of preparing the same.

### Background Art

A proton pump inhibitor (PPI) is an effective inhibitor of gastric acid secretion by inhibition of H +, K + -ATPase, an enzyme involved in the final stage of hydrogen ion production in parietal cells. In addition, a PPI is very effective in the treatment of human gastric acid-related diseases such as gastric ulcers, hemorrhagic ulcers, duodenal ulcers, non-steroidal anti-inflammatory drug (NSAID)-induced ulcers, peptic ulcers, erosive esophagitis, gastroesophageal reflux, and Helicobacter pylori infection, Zollinger-Ellison syndrome, indigestion and gastritis.

However, some proton pump inhibitors have properties such as color change or rapid decomposition upon contact with moisture, and are very unstable under acidic to neutral conditions, thus having significant constraints in preparation.

To address such problems, Patent Document 1 (Korean Patent Application Publication No.10-2013-0115593) discloses a bilayer tablet including an NSAID and a proton pump inhibitor, wherein a basic additive is added to a layer including the proton pump inhibitor to improve the stability of the proton pump inhibitor so that the production of related substances is minimized and a basic environment is created.

CN 101 711 753 A refers to a preparation method of a lansoprazole solid preparation, and solving the technical problem of providing a preparation method capable of ensuring a favorable content uniformity of the lansoprazole solid preparation. The preparation method comprises the following steps of: A, sequentially dissolving an alkaline material and the lansoproazole into anhydrous ethyl alcohol to obtain a mixed solution for later use; B, dissolving a bulking agent into the mixed solution obtained in the step A, and pelletizing; and C, preparing a solid preparation by processing particles obtained from the pelletizing according to a traditional method.

EP 2 929 885 A1 refers to a pharmaceutical composition comprising rivaroxaban or a pharmaceutically acceptable salt thereof in combination with a proton pump inhibitor for use in the anticoagulant treatment with preventing or reducing the risk of a gastrointestinal disorder.

US 2013/273171 A1 refers to a stable formulation of dexlansoprazole for treating a digestive disorder, and methods of manufacturing the same.

CN 102 772 387 A refers to a ansoprazole composition enteric capsule comprises the following materials by weight: 20-30 parts of lansoprazole, 12-18 parts of sodium bicarbonate, 15-20 parts of calcium hydrogen phosphate, 10-20 parts of magnesium hydroxide, 25-30 parts of a enteric coating agent, 60-100 parts of filler, 20-30 parts of a disintegrating agent, 20-25 parts of binder, 20-25 parts of lubricant, and 400-500 parts of 50% ethanol solution. The capsule is safe, effective, stable during storage, and highly-soluble in intestinal juice.

HE, WEI et al.: "Influences of Sodium Carbonate on Physicochemical Properties of Lansoprazole in Oesigned Multiple Coating Pellets", AAPS PharmSciTech, vol. 11, no. 3, 2010, pages 1287-1293, XP055592405.

KR 2013 0115593 A refers to a pharmaceutical composite comprises: a first layer containing non-steroidal anti-inflammatory drug (NSAID) or a pharmaceutically acceptable salt thereof; and a second agent containing proton pump inhibitors (PPIs) or a pharmaceutically acceptable salt thereof and a basic additive. The NSAID is naproxen.

KR 100 479 637 A1 refers to a process of preparing an oral formulation containing a starch layer, an enteric coating layer and a wax layer coated on the exterior of a core containing lansoprazole is provided.

KR 870 009 717 A1 refers to a vehicles for oral administration of pharmaceutically active acid labile substances prone to discolouration, containing omeprazole where the administration vehicle comprises a core containing the omeprazole together with an alkaline reacting compound or an alkaline salt of omeprazole optionally mixed with an alkaline reacting compound, adopting the form either of a number of small beads optionally forming a tablet or a tablet as such and comprising a coating made out of one or more inert reacting subcoating layers comprising tablet excipients which are soluble or rapidly disintegrating in water, or polymeric, water soluble, filmforming compounds, optionally containing pH-buffering, alkaline compounds between the alkaline reacting core and an enteric outer coating layer.

KR 101 845 665 A1 refers to an oral solid formulation composition comprising proton pump inhibitor oral solid formulation comprising the same and manufacturing method thereof

According to prior documents, the stability of drug is improved and the production of related substances can be suppressed, in a low pH environment when released in the gastrointestinal tract. However, in the case of Patent Document 1 including 2 to 10 parts by weight of a basic additive based on 1 part by weight of a proton pump inhibitor to secure the stability of the proton pump inhibitor, the size of a tablet disadvantageously increases due to the inclusion of a large amount of the basic additive. In addition, properties such as color change or rapid decomposition when contacted with moisture have still not been addressed, which leads to a problem of storage stability.

Therefore, there is a need for research on improving the stability of a proton pump inhibitor against moisture and low pH and minimizing related substances while lowering the content of a basic additive to reduce the size of an oral tablet and, thus, secure convenience in taking drugs.

### [Related Art Document]

### [Patent Document]

(Patent Document 1) KR1020130115593 A

### Disclosure

### Technical Problem

Therefore, the present disclosure has been made in view of the above problems, and it is one object of the present disclosure to provide a method of preparing an oral solid preparation capable of providing excellent production efficiency by using a wet granulation method.

### Technical Solution

In accordance with an aspect of the present disclosure and claim 1, the above and other objects can be accomplished by a method of preparing an oral solid preparation, the method including: (1) preparing a mixed powder including a proton pump inhibitor or a pharmaceutically acceptable salt thereof; (2) preparing a binding liquid including a basic additive; (3) combining the mixed powder and the binding liquid, and then drying the same; and (4) granulating a dried product obtained by the step (3) to prepare granules,
wherein a solvent for the binding liquid comprises water; and at least one organic solvent miscible with water, wherein the organic solvent is selected from ethanol, isopropanol and acetone, and
the preparing of the binding liquid comprises (a) dissolving the basic additive in water; and (b) adding the organic solvent to a solution obtained by the step (a) and mixing the same,
wherein the content of the basic additive is 2 to 13 parts by weight based on 100 parts by weight of the proton pump inhibitor or a pharmaceutically acceptable salt thereof,
wherein the mixed powder further comprises one or more selected from the group consisting of a diluent and a water-soluble polymer,
wherein the preparing of the binding liquid further comprises adding an enteric substrate to a mixed solution obtained according to the step (b) and dispersing the same.

The content of the basic additive is 2 to 13 parts by weight based on 100 parts by weight of the proton pump inhibitor or a pharmaceutically acceptable salt thereof.

The mixed powder include one or more selected from the group consisting of a diluent and a water-soluble polymer.

A solvent for the binding liquid comprises water; and at least one organic solvent miscible with water, wherein the organic solvent is selected from ethanol, isopropanol and acetone. The preparing of the binding liquid includes (a) dissolving the basic additive in water; and (b) adding the organic solvent to a solution obtained by the step (a) and mixing the same. In addition, the preparing of the binding liquid further includes adding an enteric substrate to a mixed solution obtained according to the step (b) and dispersing the same.

In the step (3), the drying may be performed at 20 to 60°C.

The method of preparing an oral solid preparation according to the present disclosure may further include mixing and tableting the granules obtained according to the step (4) with one or more selected from the group consisting of mannitol, a disintegrant, a diluent and a lubricant to prepare a tablet. In addition, the method may further include coating the tablet with a water-soluble coating layer; and coating a surface of the water-soluble coating layer with an enteric coating layer.

### Advantageous Effects

An oral solid preparation composition according to the present disclosure includes a basic additive in an appropriate content, thereby improving the stability of a proton pump inhibitor. Accordingly, an oral granule preparation allowing the minimization of the production of related substances, capable of providing improved drug stability even in a low pH environment when released in the stomach, and exhibiting excellent stability without discoloration even under moisture or humidity conditions can be provided.

In addition, since a method of preparing an oral solid preparation according to the present disclosure adopts a wet granulation method, improved production efficiency can be provided.

### Description of Drawings

FIG. 1 illustrates a schematic diagram of a process of preparing an oral solid preparation according to an embodiment of the present disclosure.
FIG. 2 includes photographs for comparing the colors of granules prepared according to Examples 1 to 3 and Comparative Examples 1 to 3.
FIG. 3 is a graph illustrating a dissolution test result of granules prepared according to Example 1.

### Modes of the Invention

The present disclosure relates to an oral solid preparation composition including a proton pump inhibitor (PPI), an oral solid preparation including the same, and a method of preparing the same.

First, the oral solid preparation composition is described in detail. The composition includes a proton pump inhibitor or a pharmaceutically acceptable salt thereof; and a binding liquid including a basic additive, wherein the content of the basic additive is 2 to 13 parts by weight based on 100 parts by weight of the proton pump inhibitor or a pharmaceutically acceptable salt thereof.

The proton pump inhibitor provides a medicinal effect by inhibiting the production of hydrochloric acid through inhibition of the proton pump (H ⁺/K ⁺ -ATPase) of parietal cells and weakening the degree of acidity in the digestive tract. In the present disclosure, the proton pump inhibitor may include one or more selected from the group consisting of dexlansoprazole, esomeprazole, lansoprazole, omeprazole, pantoprazole, rabeprazole, tenatoprazole, pharmaceutically acceptable salts thereof and precursors thereof, preferably dexlansoprazole.

The dexlansoprazole is being widely used as a therapeutic agent for peptic ulcers, is an optical isomer of lansoprazole, and a compound represented by Formula 1 below:

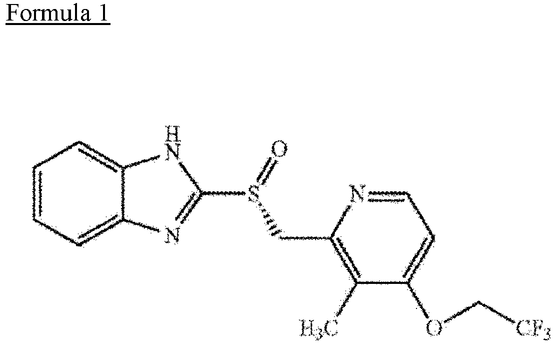

Dexlansoprazole has the property of changing color or rapidly decomposing when contacted with moisture. Such a property is also exhibited in an acidic or neutral aqueous solution. This property may cause discoloration due to moisture or humidity in a process of preparing an oral solid preparation or during storage thereof and a stability decrease due to low pH when released in the stomach.

The problems are commonly exhibited also in proton pump inhibitors such as esomeprazole other than dexlansoprazole. Accordingly, in the present disclosure, an appropriate amount of a basic additive was included together with a proton pump inhibitor so that the stability of a proton pump inhibitor was improved, thereby minimizing the influence of moisture or a low pH environment thereon. In addition, a production amount of related substances was significantly reduced.

The content of the basic additive is 2 to 13 parts by weight based on 100 parts by weight of the proton pump inhibitor or a pharmaceutically acceptable salt thereof. When the content of the basic additive was less than 2 parts by weight, drug stability was decreased in a low pH environment, and a production amount of related substances increased. On the other hand, when the content of the basic additive was greater than 13 parts by weight, a discoloration problem occurred under a moisture or humidity condition.

The basic additive may include one or more selected from the group consisting of NaOH, NaHCO₃, CaCO₃, MgCO₃, KH₂PO₄, K₂HPO₃, Ca(OH)₂, Mg(OH)₂, Na₂HPO₄, MgCO₃, NaH₂PO₄, Na₃PO₄, calcium trichloride phosphate, arginine, lysine, histidine, meglumine, aluminum magnesium silicate, aluminum magnesium metasilicate and pharmaceutically acceptable salts thereof, preferably NaOH.

The oral solid preparation composition further includes one or more selected from the group consisting of a diluent and a water-soluble polymer, and further includes an enteric substrate.

The diluent refers to a substance capable of maintaining a certain volume of granules. As the diluent, any diluent which does not affect the action of a proton pump inhibitor may be used. For example, the diluent may be one or more selected from the group consisting of mannitol, sucrose, lactose, sorbitol, xylitol and glucose, preferably lactose.

The water-soluble polymer is added to delay the release of a drug. As the water-soluble polymer, any water-soluble polymer which does not affect the action of a proton pump inhibitor may be used. For example, one or more selected from the group consisting of hydroxypropyl cellulose, hydroxypropyl methyl cellulose, hydroxymethyl cellulose, hydroxyethyl cellulose, hydroxybutyl cellulose, hydroxypentyl cellulose and hydroxypropyl butyl cellulose may be included. Preferably, hydroxypropyl methyl cellulose may be used.

The enteric substrate acts to suppress the release of a drug in the stomach. In the specification, "enteric" means a characteristic of not being disintegrated and dissolved for 2 hours in a gastric fluid condition (near pH 1.2), but being rapidly disintegrated and dissolved within 1 hour in an intestinal fluid condition (near pH 7.2). As the enteric substrate, any enteric substrate which does not affect the action of active ingredients may be used. For example, one or more selected from the group consisting of a poly(methacrylic acid, methyl methacrylate) copolymer (e.g., Eudragit L, Eudragit S, manufactured by Evonik, Germany), hydroxypropyl methylcellulose acetate succinate, hydroxypropyl methylcellulose phthalate and carboxymethylethylcellulose may be used. Preferably, a poly(methacrylic acid, methyl methacrylate) copolymer may be used.

Meanwhile, the present disclosure provides an oral solid preparation including the oral solid preparation composition. The oral solid preparation may be any one formulation selected from the group consisting of a granule, a pellet, a tablet and a capsule.

The oral solid preparation may further include one or more selected from the group consisting of mannitol, a disintegrant, a diluent and a lubricant. For example, when a formulation of the oral solid preparation according to the present disclosure is a tablet, granules prepared from the oral solid preparation composition may further include one or more selected from the group consisting of mannitol, a disintegrant, a diluent and a lubricant.

The mannitol serves to increase flowability of the oral solid preparation composition. When mannitol is included, an oral solid preparation composition having excellent mixing uniformity and fluidity may be provided.

The disintegrant serves to facilitate the collapse or disintegration of a solid formulation after oral administration. The disintegrant may include one or more selected from the group consisting of microcrystalline cellulose, low-substituted hydroxypropyl cellulose, sodium croscarmellose, sodium starch glycolate, sodium carboxymethyl cellulose, calcium carboxymethyl cellulose and crospovidone.

The diluent is additionally added to the oral solid preparation prepared using the oral solid preparation composition, and types thereof have been described above.

The lubricant serves to improve the fluidity of amorphous dexransoprazole particles, prevent inter-particle friction, and prevent amorphous dextransoprazole particles from adhering to a tablet machine. The lubricant may include one or more selected from the group consisting of magnesium stearate, stearic acid, zinc stearate, calcium stearate, talc, sodium stearyl fumarate, talc, silicon dioxide and colloidal silicon dioxide.

The oral solid preparation provided according to the present disclosure may further include a core including the oral solid preparation composition, a water-soluble coating layer covering a surface of the core, and an enteric coating layer covering a surface of the water-soluble coating layer. When the oral solid preparation is designed as a double coating preparation in such a form, acid resistance may be secured so that drug release in the stomach may be suppressed and drug release in the intestines may be promoted. Accordingly, patient administration compliance may be improved.

The enteric coating layer is an outermost coating layer of the oral solid preparation according to the present disclosure and serves to suppress the release of a drug in the stomach. Such an enteric coating layer may include one or more selected from the group consisting of hydroxypropyl methyl cellulose phthalate (HPMCP), hydroxypropyl methyl cellulose acetate succinate (HPMCAS), cellulose acetate phthalate, and derivatives thereof.

The water-soluble coating layer is a coating layer present between the core including the oral solid preparation composition and the enteric coating layer, allows the enteric coating layer to be effectively and stably coated, and serves to minimize influence of pH on a proton pump inhibitor upon coating of the enteric coating layer. Such a water-soluble coating layer may include one or more selected from the group consisting of cellulose ether, polyvinylpyrrolidone, and polyvinyl alcohol. Here, the cellulose ether may include one or more selected from the group consisting of hydroxyalkyl cellulose, hydroxyalkyl alkyl cellulose, preferably one or more selected from the group consisting of hydroxypropyl cellulose, hydroxypropyl methyl cellulose, hydroxymethyl cellulose, hydroxyethyl cellulose, hydroxybutyl cellulose, hydroxypentyl cellulose and hydroxypropyl butyl cellulose.

In addition, the present disclosure provides the method of preparing an oral solid preparation, the method including (1) preparing a mixed powder including a proton pump inhibitor or a pharmaceutically acceptable salt thereof; (2) preparing a binding liquid including a basic additive; (3) combining the mixed powder and the binding liquid, and then drying the same; and (4) granulating a dried product obtained by the step (3) to prepare granules.

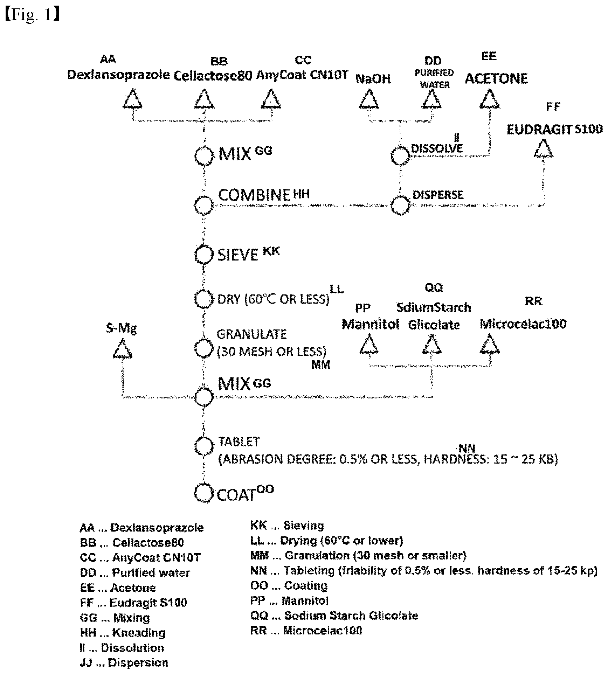

Hereinafter, a method of preparing an oral solid preparation according to an embodiment of the present disclosure is described in detail with reference to FIG. 1.

### (1) Mixed powder preparation step

This step is a step of preparing a mixed powder including a proton pump inhibitor or a pharmaceutically acceptable salt thereof. Here, the mixed powder further includes one or more selected from the group consisting of a diluent and a water-soluble polymer. In addition, the types of the proton pump inhibitor, the diluent and the water-soluble polymer used in the present disclosure have been described above.

This step may include a process of sieving the proton pump inhibitor or a pharmaceutically acceptable salt thereof and an additional additive (e.g., a diluent and/or a water-soluble polymer) during preparation of the mixed powder. The sieving process is preferred because a mixed powder having a desired particle size in a uniformly mixed state may be provided through the sieving process.

### (2) Binding liquid preparation step

This step is a step of preparing a binding liquid including a basic additive. The content of the basic additive is 2 to 13 parts by weight based on 100 parts by weight of the proton pump inhibitor or a pharmaceutically acceptable salt thereof. The types of the basic additive have been described above.

The oral solid preparation according to the present disclosure is prepared by a wet granulation method. According to a general wet granulation method, a mixture of an active ingredient, such as a proton pump inhibitor, and an additive is combined with a binding liquid, followed by a drying process, etc. In such a conventional wet granulation method, a basic additive included in the mixture is combined with a binding liquid. In this case, the basic additive should be added in a larger amount than a proton pump inhibitor to obtain a solid preparation having desired stability. However, when a large amount of basic additive is included as described above, the size of a tablet to be finally ingested increases, which causes a problem of discomfort in taking drugs. As a result of diligent research to solve this problem, the present inventors confirmed that a binding liquid applicable to a wet granulation method may be prepared using only a small amount of basic additive when granules are prepared, after preparing a binding liquid including a basic additive, through a process of combining the mixed powder prepared according to the step (1) with the binding liquid and drying the same, so that an oral solid preparation having excellent stability may be prepared, thus completing the present disclosure.

A solvent for preparing the binding liquid comprises water; and at least one organic solvent miscible with water. For example, the organic solvent is selected from ethanol, isopropanol and acetone, preferably acetone having a boiling point of 60°C or less, because a proton pump inhibitor such as dexlansoprazole is sensitive to temperature. For example, granules including dexlansoprazole may be discolored when dried at high temperature. Accordingly, an organic solvent that has a low boiling point and is applicable to manufacture of pharmaceuticals is preferred. However, since the basic additive used in the present disclosure is not dissolved in the organic solvent, a binding liquid may be prepared through the following steps.

In particular, the step of preparing the binding liquid includes (a) dissolving the basic additive in water, and (b) adding the organic solvent to a solution obtained by the step (a) and mixing the same. In addition, the step of preparing the binding liquid further includes a step of adding an enteric substrate to a mixed solution obtained according to the step (b) and dispersing the same. Here, the types of enteric substrate used have been described above.

### (3) Combination and drying step

This step is a step of combining the mixed powder obtained by the step (1) with the binding liquid obtained by the step (2), and then drying the same. Here, the combining refers to a process of mixing the binding liquid with the mixed powder to prepare wet granules.

Wet granules formed by combining the mixed powder with the binding liquid may be dried through a sieving step. Here, the sieving step refers to a process of filtering the wet granules through a sieve to sort particles having a predetermined size or less.

The drying process may be performed in a temperature range of 20 to 60°C, preferably 50°C or less, more preferably 40°C or less, most preferably 20°C to 40°C, in consideration of the stability of the proton pump inhibitor using air drying, fluid bed drying, oven drying or microwave drying.

### (4) Granule preparation step

This step is a step of granulating the dried product obtained by the step (3) to prepare granules. The granulation is a process of making the size of granules uniform. In this step, a generally used granulator may be used without limitation. The size of granulated granule particles is preferably 30 mesh or less to provide an oral tablet having a low abrasion degree when subjected to a subsequent tableting process.

The method of preparing an oral solid preparation according to the present disclosure may further include a step of mixing the granules obtained according to the step (4) with one or more selected from the group consisting of mannitol, a disintegrant, a diluent and a lubricant and tableting the same to prepare a tablet. The hardness of the tableted tablet may be 98.1 to 245.2 N (10 to 25 kp). An oral tablet tableted in such a manner may exhibit an abrasion degree of 0.5% or less.

In addition, the method of preparing an oral solid preparation according to the present disclosure may further include a step of coating the tableted tablet with a water-soluble coating layer; and a step of coating a surface of the water-soluble coating layer with an enteric coating layer. Accordingly, a double-coated oral tablet may be prepared, thereby securing the acid resistance of the tablet and increasing patient administration compliance.

Hereinafter, the present disclosure will be described in more detail with reference to the following examples, but the present disclosure is not limited to the examples.

### Example 1

100 parts by weight of dexlansoprazole, 108.3 parts by weight of a diluent, and 166.7 parts by weight of a water-soluble polymer were mixed to prepare a mixed powder.

Next, 5.8 parts by weight of sodium hydroxide (NaOH), as a basic additive, was dissolved in 58.3 parts by weight of purified water and 50 parts by weight of acetone was added thereto, followed by mixing. In the mixed solution, 25 parts by weight of an enteric substrate was dispersed, thereby preparing a binding liquid.

Next, the binding liquid was added to and mixed with the mixed powder, followed by drying at 55°C for 2 hours through a sieving process. In the sieving process, a wet powder was screened using a 14-mesh sieve.

Next, a dried product obtained through the drying process was granulated to prepare granules. Here, granules having a particle size of 30 mesh or less were screened through the granulation process.

### Examples 2 and 3 and Comparative Examples 1 to 3

Granules were prepared in the same manner as in Example 1 except that the contents of dexlansoprazole, a diluent, a water-soluble polymer, a basic additive, an enteric substrate, purified water and acetone were adjusted as summarized in Table 1 below. Here, a content unit of each component shown in the following Table 1 is parts by weight.

**[Table 1]**

| | Example 1 | Example 2 | Example 3 | Comparative Example 1 | Comparative Example 2 | Comparative Example 3 |
|---|---|---|---|---|---|---|
| Dexlansoprazole | 100.0 | 100.0 | 100.0 | 1100.0 | 100 | 1100 |
| Diluent | 108.3 | 108.3 | 108.3 | 1140.0 | 140 | 140 |
| Water-soluble polymer | 166.7 | 166.7 | 166.7 | 170.0 | 170 | 170 |
| Basic additive | 5.8 | 8.3 | 12.5 | 1.0 | 15.0 | - |
| Enteric substrate | 25.0 | 25.0 | 125.0 | 25.0 | 25.0 | 25.0 |
| Purified water | 58.3 | 58.3 | 58.3 | 58.0 | 58.0 | 58.0 |
| Acetone | 50.0 | 50.0 | 150.0 | 50.0 | 50.0 | 50.0 |

| | | | | | | |
|---|---|---|---|---|---|---|
| * PPI: dexlansoprazole (Dexlansoprazole Amorphous, Amino Chemicals) * Diluent: lactose (Cellactose 80, MEGGLE Co., Ltd.) * Water-soluble polymer : hydroxypropyl methyl cellulose (AnyCoat CN101T, LOTTE Fine Chemical) * Basic additive: sodium hydroxide (NaOH, MERCK) * Enteric substrate: poly(methacrylic acid, methyl methacrylate) copolymer(Eudragit S100, Evonik) * Organic solvent: acetone (Acetone, DAEJUNG CHEMICAL & METALS CO., LTD.) | | | | | | |

### <Evaluation methods>

### 1. Related substances (%)

The granules prepared according to Examples 1 to 3 and Comparative Examples 1 to 3 were subjected to a stability test. In particular, the contents of related substance B (Imp.B), related substance C (Imp.C) and Dex N-OX sulphoxide, as representative degradation products of dexlansoprazole, and the content of a total of the related substances were measured using liquid chromatography (UPLC). Results are summarized in Table 2 below. Here, the Dex N-OX sulphoxide was 2-[3-methyl-1-oxy-4-(2,2,2-trifluoro-ethoxy)-pyridin-2-ylmethanesulfinyl]-1H-benzoimidazole as a related substance of dexlansoprazole.

HPLC analysis conditions for the related substances were as follows:
- Column: X-Bridge Shield RP18 250 mm x 4.6 mm, 5 µm
- Injection amount: 20 µl
- Detector: UV absorbance spectrometer (wavelength: 285 nm)
- Column temperature: 35 °C
- Mobile phase A: ammonium acetate 0.02 M, mobile phase B: 100 % acetonitrile
   Start: mobile phase A : mobile phase B = 80 : 20
   25 minutes: mobile phase A : mobile phase B = 25 : 75
   35 minutes: mobile phase A : mobile phase B = 25 : 75
- Diluent: methanol/sodium hydroxide 0.01 M (25:75)

### 2. Dissolution test

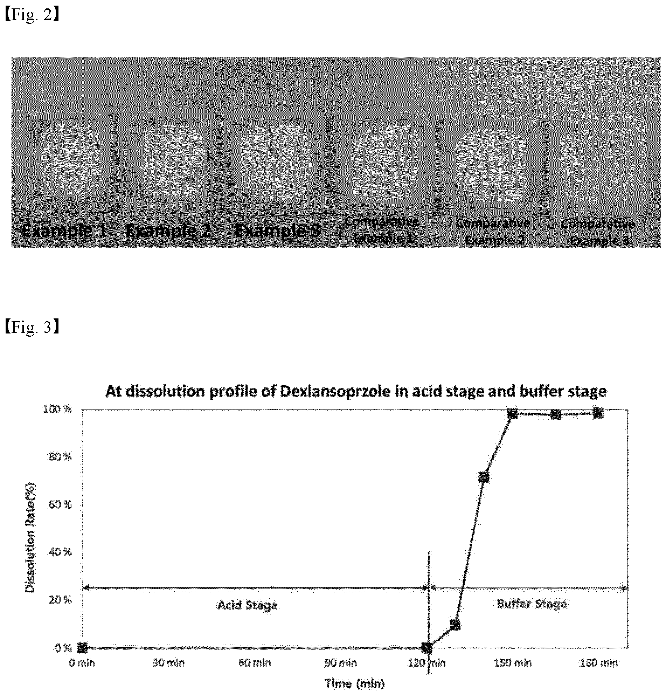

A dissolution rate of granules prepared according to Example 1 was evaluated under the following conditions. A result is illustrated in FIG. 3.
- Dissolution method: Dissolution method No. 2 of the Korean Pharmacopoeia (paddle method)
- Dissolution tester type: Manufacturer - ERWEKA GmbH, type - DT 1420
- Dissolution liquid type: pH1.2(acid stage), pH7.2(buffer stage)
- Amount of dissolution liquid: 900 mL
- Temperature of dissolution liquid: 37°C
- Paddle speed: 100 rpm

Here, the dissolution test was started in an acid solution at pH1.2. After 120 minutes, the acid solution was changed to a pH 7.2 solution containing 5 mM sodium lauryl sulfate, and then the dissolution test was continued for a total of 210 minutes.

**[Table 2]**

| | Example 1 | Example 2 | Example 3 | Comparative Example 1 | Comparative Example 2 | Comparative Example 3 |
|---|---|---|---|---|---|---|
| Imp.B (%) | 0.001 | 0.001 | 0.0007 | 0.012 | 0.0004 | 0.01 |
| Imp.C (%) | 0.0008 | 0.001 | 10.0006 | 0.007 | 0.001 | 0.15 |
| Dex N-OX sulphoxide (%) | 0.0002 | 0.0001 | 0.0001 | 0.15 | 0.0001 | 0.35 |
| Total Imp. (%) | 0.035 | 0.03 | 0.028 | 0.35 | 0.031 | 0.54 |

Examining Table 1, a related-substance production rate in the granules prepared according to Comparative Example 2, in which the content of a basic additive (NaOH) was 15 parts by weight based on 100 parts by weight of dexlansoprazole, was similar to those in the granules prepared according to Examples 1 to 3 in which the content of a basic additive (NaOH) was 2 to 13. However, it can be confirmed from FIG. 2 including photographs of the granules prepared according to Examples 1 to 3 and Comparative Examples 1 to 3 that the granules prepared according to Comparative Example 2 had turned yellow, compared to the granules prepared according to Examples 1 to 3. This is because dexlansoprazole was discolored to yellow due to moisture included in the binding liquid. This confirmed, in the case of the granules prepared according to Comparative Example 2, that moisture stability was decreased. In addition, it was confirmed that in the case of the granules prepared according to Examples 1 to 3, a related-substance production rate was significantly reduced, compared to the granules prepared according to Comparative Examples 1 and 3 including a basic additive in an amount less than a lower limit of the range. Meanwhile, the granules of Comparative Example 3 exhibited a reddish color as shown in FIG. 2. This color appears when related substances are included in a large amount. It can be confirmed only through visual observation that a related-substance production rate greatly increases in the case of the granules prepared using a binding liquid excluding a basic additive.

In addition, FIG. 3 is a graph illustrating a dissolution test result of the granules prepared according to Example 1. Examining FIG. 3, it can be confirmed that, in the case of the granules prepared according to Example 1, dissolution is suppressed in an acid stage (pH 1.2) and a dissolution rate greatly increases in a buffer stage (pH 7.2). From this, it can be confirmed that, in the case of the oral solid preparation prepared according to the present disclosure, dissolution of dexlansoprazole is suppressed in the stomach and promoted in the intestines, so that efficient delivery to the body can be accomplished.

## Claims

1. A method of preparing an oral solid preparation, the method comprising:
(1) preparing a mixed powder comprising a proton pump inhibitor or a pharmaceutically acceptable salt thereof;
(2) preparing a binding liquid comprising a basic additive;
(3) combining the mixed powder and the binding liquid, and then drying the same; and
(4) granulating a dried product obtained by the step (3) to prepare granules,
wherein a solvent for the binding liquid comprises water; and at least one organic solvent miscible with water, wherein the organic solvent is selected from ethanol, isopropanol and acetone, and
the preparing of the binding liquid comprises (a) dissolving the basic additive in water; and (b) adding the organic solvent to a solution obtained by the step (a) and mixing the same,
wherein the content of the basic additive is 2 to 13 parts by weight based on 100 parts by weight of the proton pump inhibitor or a pharmaceutically acceptable salt thereof,
wherein the mixed powder further comprises one or more selected from the group consisting of a diluent and a water-soluble polymer,
wherein the preparing of the binding liquid further comprises adding an enteric substrate to a mixed solution obtained according to the step (b) and dispersing the same.

2. The method according to claim 1, wherein, in the step (3) the drying is performed at 20 to 60°C.

3. The method according to claim 1, further comprising mixing and tableting the granules obtained according to the step (4) with one or more selected from the group consisting of mannitol, a disintegrant, a diluent and a lubricant to prepare a tablet.

4. The method according to claim 3, further comprising coating the tablet with a water-soluble coating layer; and coating a surface of the water-soluble coating layer with an enteric coating layer.

## Patentansprüche

1. Verfahren zur Herstellung einer oralen festen Zubereitung, wobei das Verfahren Folgendes umfasst:
(1) die Herstellung einer Pulvermischung, die einen Protonenpumpenhemmer oder ein pharmazeutisch unbedenkliches Salz davon umfasst,
(2) die Herstellung einer Bindungsflüssigkeit, die einen basischen Zusatzstoff umfasst,
(3) das Zusammengeben der Pulvermischung mit der Bindungsflüssigkeit, und dann das Trocknen davon, und
(4) das Granulieren eines in Schritt (3) erhaltenen getrockneten Produkts unter Bildung eines Granulats,
wobei ein Lösungsmittel für die Bindungsflüssigkeit Wasser und mindestens ein mit Wasser mischbares organisches Lösungsmittel umfasst, wobei das organische Lösungsmittel aus Ethanol, Isopropanol und Aceton ausgewählt ist, und
wobei die Herstellung der Bindungsflüssigkeit Folgendes umfasst: (a) das Lösen des basischen Zusatzstoffs in Wasser und (b) die Zugabe des organischen Lösungsmittels zu einer in Schritt (a) erhaltenen Lösung und das Mischen davon, wobei der Gehalt des basischen Zusatzstoffs 2 bis 13 Gewichtsteile beträgt, bezogen auf 100 Gewichtsteile des Protonenpumpenhemmers oder eines pharmazeutisch unbedenklichen Salzes davon,
wobei die Pulvermischung weiterhin eine oder mehrere Komponenten ausgewählt aus der aus einem Verdünnungsmittel und einem wasserlöslichen Polymer bestehenden Gruppe umfasst,
wobei die Herstellung der Bindungsflüssigkeit weiterhin die Zugabe eines magensaftresistenten Substrats zu einer gemäß Schritt (b) erhaltenen gemischten Lösung und das Dispergieren davon umfasst.

2. Verfahren nach Anspruch 1, wobei die Trocknung in Schritt (3) bei 20 bis 60 °C erfolgt.

3. Verfahren nach Anspruch 1, weiterhin umfassend das Mischen und Tablettieren des gemäß Schritt (4) erhaltenen Granulats mit einem oder mehreren Komponenten ausgewählt aus der aus Mannit, einem Sprengmittel, einem Verdünnungsmittel und einem Schmiermittel bestehenden Gruppe, unter Bildung einer Tablette.

4. Verfahren nach Anspruch 3, weiterhin umfassend das Beschichten der Tablette mit einer wasserlöslichen Überzugsschicht und das Beschichten der wasserlöslichen Überzugsschicht mit einer magensaftresistenten Überzugsschicht.

## Revendications

1. Procédé de préparation d'une préparation solide orale, le procédé comprenant :
(1) la préparation d'une poudre mélangée comprenant un inhibiteur de pompe à protons ou un sel pharmaceutiquement acceptable correspondant ;
(2) la préparation d'un liquide liant comprenant un additif basique ;
(3) la combinaison de la poudre mélangée et du liquide liant, et ensuite leur séchage ; et
(4) la granulation d'un produit séché obtenu par l'étape (3) pour préparer des granules,
un solvant pour le liquide liant comprenant de l'eau ; et au moins un solvant organique miscible avec l'eau, le solvant organique étant choisi parmi l'éthanol, l'isopropanol et l'acétone, et
la préparation du liquide liant comprenant (a) la dissolution de l'additif basique dans de l'eau ; et (b) l'ajout du solvant organique à une solution obtenue par l'étape (a) et leur mélange,
la teneur de l'additif basique étant de 2 à 13 parties en poids sur la base de 100 parties en poids de l'inhibiteur de pompe à protons ou d'un sel pharmaceutiquement acceptable correspondant,
la poudre mélangée comprenant en outre l'un ou plusieurs choisis dans le groupe constitué par un diluant et un polymère soluble dans l'eau,
la préparation du liquide liant comprenant en outre l'ajout d'un substrat entérique à une solution mélangée obtenue selon l'étape (b) et leur dispersion.

2. Procédé selon la revendication 1, dans l'étape (3), le séchage étant réalisé à une température de 20 à 60 °C.

3. Procédé selon la revendication 1, comprenant en outre le mélange et la mise en comprimé des granules obtenus selon l'étape (4) avec l'un ou plusieurs choisis dans le groupe constitué par le mannitol, un désintégrant, un diluant et un lubrifiant pour préparer un comprimé.

4. Procédé selon la revendication 3, comprenant en outre le revêtement du comprimé avec une couche de revêtement soluble dans l'eau ; et le revêtement d'une surface de la couche de revêtement soluble dans l'eau par une couche de revêtement entérique.
